(19) **Europäisches Patentamt European Patent Office Office européen des brevets**

(11) **EP 4 549 547 A1**

(12) **EUROPEAN PATENT APPLICATION**
published in accordance with Art. 153(4) EPC

(43) Date of publication:
07.05.2025  Bulletin 2025/19

(21) Application number: 23828663.7

(22) Date of filing: 28.04.2023

(51) International Patent Classification (IPC):
*C12N 1/20* (2006.01)          *C12Q 1/06* (2006.01)
*G01N 27/48* (2006.01)          *C12M 1/34* (2006.01)

(52) Cooperative Patent Classification (CPC):
C12M 1/34; C12N 1/20; C12Q 1/06; G01N 27/48

(86) International application number:
PCT/JP2023/016896

(87) International publication number:
WO 2024/004371 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority:  28.06.2022  JP 2022103822

(71) Applicant: University Public Corporation Osaka
Osaka-shi, Osaka 5360025 (JP)

(72) Inventors:
• SHIIGI,Hiroshi
Sakai-shi, Osaka 599-8531 (JP)

• IKEDA,Hikaru
Sakai-shi, Osaka 599-8531 (JP)
• YAMAMOTO,Yojiro
Sakai-shi, Osaka 599-8531 (JP)
• TOKONAMI,Akira
Sakai-shi, Osaka 599-8531 (JP)
• NISHII,Shigeki
Sakai-shi, Osaka 599-8531 (JP)

(74) Representative: Viering, Jentschura & Partner
mbB
Patent- und Rechtsanwälte
Am Brauhaus 8
01099 Dresden (DE)

(54) **LIVE BACTERIA QUANTIFYING DEVICE, QUANTIFYING METHOD, AND KIT FOR QUANTIFYING LIVE BACTERIA**

(57)     A live bacteria quantifying method includes: a deposition step in which live bacteria are placed in a measurement vessel and are mixed for a predetermined period of time, the measurement vessel containing an oxidation-reduction substance which is cell-membrane-permeable for the live bacteria as a measurement target, is oxidized or reduced in cells, and is deposited in the cells; a current peak measurement step in which after the deposition step, electrochemical measurement is performed for a measurement solution and a current peak of a residual oxidation-reduction substance is obtained; and a quantification evaluation step in which a current peak difference between the current peak of the residual oxidation-reduction substance, the current peak being measured in the current peak measurement step, and a current peak of an initial oxidation-reduction substance, the current peak being measured in advance, is obtained and quantification evaluation of the live bacteria is performed based on the current peak difference.

EP 4 549 547 A1

Fig1

| | |
|---|---|
| 103 display unit | 12 current peak measurement unit |
| 104 input operation unit | 121 voltage application control unit |
| 105 control unit | 122 current response measurement unit |
| 14 memory | 13 quantification evaluation unit |
| 15 data saving unit | |
| 109 | 102 |

1

20

22

23

24

E

**Description**

Technical Field

The present invention relates to a live bacteria quantifying device, a quantifying method, and a kit for quantifying live bacteria.

Background Art

As a method of quantifying live bacteria (bacteria) with a small size of approximately several $\mu$m, for example, evaluation by a culturing method of performing a count of colonies formed by proliferation of a single cell on a flat plate culture medium and evaluation by microscopy using fluorescent labeling have been widely used. The culturing method requires a culturing time of one day or longer. The culturing method and microscopy require skillful techniques and expensive devices.

Further, there is also evaluation that is based on spectroscopic and electrochemical measurement focusing on dissolved oxygen which is consumed by aerobic respiration by bacteria. This measurement method focusing on respiration is simple but has difficulty in absolute evaluation because oxygen solubility differs depending on environments.

Further, there is another method in which cell-membrane-permeable MTT (one kind of tetrazolium salt, 3-(4,5-di-methylthiazol-2-yl)-2,5-diphenyl tetrazolium bromide), as a formazan dye is caused to be incorporated in cells of live bacteria, the bacteria are cultured and discolored (yellow to violet), and a spectral absorption wavelength is thereafter measured. This method usually requires culture for five hours or longer.

Patent Literature 1 discloses that in a method for detecting bacteria, a mediator, which causes a reaction in response to an action of an enzyme present in a cell membrane of bacteria which are present in a specimen and are to be detected, is caused to react with the enzyme, the reacting mediator is detected or quantified by an electrochemical method, and the bacteria are thereby detected or quantified. Examples of the mediator may include benzoquinone, 2,6-dimethylbenzoquinone, methoxybenzoquinone, vitamin K3 (menadione), and so forth.

Non Patent Literature 1 discloses a method in which bacteria are caused to incorporate the cell-membrane-permeable MTT in their cells and are cultured, the cell membranes are destroyed on an electrode, MTT formazan (the reduced and discolored MTT may be referred to as "MTT formazan") in the cells is electrochemically measured. In this method, operations are needed in which the bacteria in a measurement solution are centrifuged, only the bacteria are transferred onto an electrode with a large area, the cell membranes are destroyed, and the bacteria are dried on the electrode, and this method has many operation procedures and is complicated.

Citation List

Patent Literature

Patent Literature 1: Japanese Patent No. 4448223 Non Patent Literature

Non Patent Literature 1: Authors "Kengo Ishiki, Dung Q. Nguyen, Aya Morishita, Hiroshi Shiigi, and Tsutomu Nagaoka", "Electrochemical Detection of Viable Bacterial Cells Using a Tetrazolium Salt", Analytical Chemistry 2018, Vol. 90, pp. 10,903-10,909.

Summary of Invention

Technical Problem

The present invention provides a live bacteria quantifying device, a quantifying method, and a kit for quantifying live bacteria that can, more simply than related art, perform electrochemical evaluation of bacteria activity by using an oxidation-reduction substance (for example, an artificial dye).

Solution to Problem

A first live bacteria quantifying device includes:

a data accumulation unit that accumulates first or second collating data including a current peak difference ($\Delta I_p$) which is set in advance and the number of live bacteria which corresponds to the current peak difference ($\Delta I_p$) ;
a current peak measurement unit that performs electrochemical measurement (sweeping from a positive-side potential to a negative-side potential or from a negative-side potential to a positive-side potential) about a measurement solution containing an oxidation-reduction substance, which is cell-membrane-permeable for live bacteria as a measurement target, is oxidized or reduced in cells, and is deposited in the cells of the live bacteria, and that obtains at least one current peak ($I_{p1}$) of a residual oxidation-reduction substance;

a quantification evaluation unit that obtains a current peak difference ($\Delta I_p$) between the current peak ($I_{p1}$) of the residual oxidation-reduction substance, the current peak ($I_{p1}$) being obtained by measuring the measurement solution, in which deposition in the live bacteria is already performed, by the current peak measurement unit, and a current peak ($I_{p0}$) of an initial oxidation-reduction substance which is not yet deposited in the live bacteria, the current peak ($I_{p0}$) corresponding to the at least one current peak ($I_{p1}$) and being set in advance (being acquired via input means or communication means, being stored in the data accumulation unit, or being measured in advance by the current peak measurement unit in a state the live bacteria is not yet brought into contact with the oxidation-reduction substance, for example), that performs collation with first or second collation data of the data accumulation unit by the current peak difference ($\Delta I_p$) (as the current peak difference ($\Delta I_p$) as a search key), and that performs quantification evaluation of the live bacteria.

The "measurement solution containing the residual oxidation-reduction substance" is (1) a measurement solution which results from a process in which after live bacteria are placed in a measurement vessel containing an oxidation-reduction substance, which is cell-membrane-permeable for the live bacteria as a measurement target, is oxidized or reduced in cells, and is deposited (or insolubilized) in the cells, mixing is performed for a predetermined period of time (for example, 20, 30, or 40 minutes), and the oxidation-reduction substance is deposited, (2) a supernatant liquid in which the above measurement solution resulting from deposition has been centrifuged, and (3) a measurement solution in a suspended state where the measurement solution contains the live bacteria and the residual oxidation-reduction substance (a suspension in a mixed state or a suspension in a state where mixing is being performed).

A second live bacteria quantifying device includes:

a data accumulation unit that accumulates first or second collating data including a current peak ($I_{p1}$) of a residual oxidation-reduction substance, the current peak ($I_{p1}$) being set in advance, and the number of live bacteria which corresponds to the current peak ($I_{p1}$) of the residual oxidation-reduction substance;

a current peak measurement unit that performs electrochemical measurement (sweeping at voltages in a predetermined range (from a positive-side potential to a negative-side potential or from a negative-side potential to a positive-side potential)) about a measurement solution containing the residual oxidation-reduction substance, which is not incorporated in live bacteria, and that obtains the current peak ($I_{p1}$) of the residual oxidation-reduction substance; and

a quantification evaluation unit that performs collation with first or second collation data of the data accumulation unit by using, as a key, the current peak ($I_{p1}$) of the residual oxidation-reduction substance, the current peak ($I_{p1}$) being measured by the current peal measurement unit, and that performs quantification evaluation of the live bacteria.

Because the current peak of the oxidation-reduction substance, which is initially contained in the measurement solution, is known in advance, it becomes possible to perform quantification evaluation of the number of live bacteria based on the current peak ($I_{p1}$) of the residual oxidation-reduction substance.

In each of the first and second live bacteria quantifying devices, at least one or more current peaks are obtained for each of the initial and residual oxidation-reduction substances. Further, the current peak of the initial oxidation-reduction substance may be set in accordance with a predetermined mixing time (deposition time). For example, in a case where the mixing (deposition) time is 10 minutes, the current peak of the initial oxidation-reduction substance for 10 minutes may be selected, and in a case where the mixing (deposition) time is 30 minutes, the current peak of the initial oxidation-reduction substance for 30 minutes may be selected. Further, the number of live bacteria which is obtained by quantification evaluation may be represented by a digit order. Further, as for the number of live bacteria to be quantified, the live bacteria may include a plurality of kinds of bacteria. In this case, the plurality of kinds of bacteria are quantified at the same time.

Each of the first and second live bacteria quantifying devices may include:

a data saving step of saving, in a storage medium, evaluation results obtained by the quantification evaluation unit (for example, the number of live bacteria, the current peak difference ($\Delta I_p$), and the current peak ($I_{p1}$) of the residual oxidation-reduction substance); and

an output unit that outputs the evaluation results obtained by the quantification evaluation unit or the evaluation results saved in the storage medium to output means.

Examples of "output" may include displaying the evaluation results on display means, outputting the evaluation results to a printer and printing those, transmitting the evaluation results to an external device, saving the evaluation results in the storage medium, and so forth. It is sufficient that the external device or the storage medium is connected with a local area network or the Internet.

Each of the first and second live bacteria quantifying devices may be of a small-sized portable type.

Each of the first and second live bacteria quantifying devices may have an electrode chip to which the oxidation-reduction substance of an amount set in advance is adhered. The electrode chip may be of a disposable type.

The current peak measurement unit of each of the first and second live bacteria quantifying devices may perform electrochemical measurement (sweeping at voltages in a predetermined range (from a positive-side potential to a negative-side potential or from a negative-side potential to a positive-side potential)) about the measurement solution containing the residual oxidation-reduction substance, which is not incorporated in the live bacteria, after the electrode chip is immersed in the measurement solution and may obtain the current peak ($I_{p1}$) of the residual oxidation-reduction substance.

In each of the first and second live bacteria quantifying devices, the current peak measurement unit may perform electrochemical measurement (at least sweeping at voltages in a predetermined range (from a positive-side potential to a negative-side potential)) about the measurement solution containing the residual oxidation-reduction substance, which is not incorporated in the live bacteria, and may obtain the current peak ($I_{p1}$) of the residual oxidation-reduction substance. In other words, the current peak may be obtained from a reduction current.

In each of the first and second live bacteria quantifying devices, the current peak measurement unit may perform electrochemical measurement (at least sweeping at voltages in a predetermined range (from a negative-side potential to a positive-side potential)) about the measurement solution containing the residual oxidation-reduction substance, which is not incorporated in the live bacteria, and may obtain the current peak ($I_{p1}$) of the residual oxidation-reduction substance. In other words, the current peak may be obtained from an oxidation current.

One kind or two or more kinds of current peaks may be obtained.

A first live bacteria quantifying method includes:

a deposition step in which live bacteria are placed in a measurement solution and are mixed for a predetermined period of time (for example, 20, 30, or 40 minutes), the measurement solution containing an oxidation-reduction substance which is cell-membrane-permeable for the live bacteria as a measurement target, is oxidized or reduced in cells, and is deposited (or insolubilized) in the cells of the live bacteria, and the oxidation-reduction substance is caused to be incorporated and deposited in the cells of the live bacteria;

a current peak measurement step in which after the deposition step, electrochemical measurement (sweeping at voltages in a predetermined range (from a positive-side potential to a negative-side potential or from a negative-side potential to a positive-side potential)) is performed for the measurement solution (which contains the live bacteria and a residual oxidation-reduction substance and is in a suspended state), and at least one current peak ($I_{p1}$) of the residual oxidation-reduction substance remaining in the measurement solution is obtained;

a first quantification evaluation step in which a current peak difference ($\Delta I_p$) between at least one current peak ($I_{p1}$) of the residual oxidation-reduction substance, the current peak ($I_{p1}$) being measured in the current peak measurement step, and a current peak ($I_{p0}$) of an initial oxidation-reduction substance, the current peak ($I_{p0}$) corresponding to the at least one current peak ($I_{p1}$) and being set in advance, is obtained and quantification evaluation of the live bacteria is performed based on the current peak difference ($\Delta I_p$); and/or a second quantification evaluation unit which performs quantification evaluation of the live bacteria based on the current peak ($I_{p1}$).

The number of live bacteria which corresponds to the current peak difference ($\Delta I_p$) or the current peak ($I_{p1}$) may be set in advance. Data (collating data) of the number of live bacteria which corresponds to the current peak difference ($\Delta I_p$) or the current peak ($I_{p1}$) may be saved in a storage medium. The number of live bacteria may be quantified by performing collation with first or second collating data by using, as a key, the current peak difference ($\Delta I_p$) or the current peak ($I_{p1}$).

The current peak measurement step may include: a voltage application step of applying voltages in a predetermined range (from a positive-side potential to a negative-side potential or from a negative-side potential to a positive-side potential) according to the electrochemical measurement to the electrode chip or the action electrode and the reference electrode, which are formed as the single electrode; and

a current response measurement step of obtaining a current peak (peak height) of a current response which is measured by applying the voltages in the predetermined range (and a potential (peak potential) at the current peak).

The current peak of the initial oxidation-reduction substance may be set in accordance with a predetermined mixing time (deposition time). For example, in a case where the mixing (deposition) time is 10 minutes, the current peak of the initial oxidation-reduction substance for 10 minutes may be selected, and in a case where the mixing (deposition) time is 30 minutes, the current peak of the initial oxidation-reduction substance for 30 minutes may be selected.

The number of live bacteria which is obtained by quantification evaluation may be represented by a digit order.

As for the number of live bacteria to be quantified, the live bacteria may include a plurality of kinds of bacteria. In this case, the plurality of kinds of bacteria are quantified at the same time.

In the current peak measurement step (or the current response measurement step), electrochemical measurement (at least sweeping at voltages in a predetermined range (from a positive-side potential to a negative-side potential)) may be performed for the measurement solution containing the residual oxidation-reduction substance, which is not incorporated in the live bacteria, and the current peak ($I_{p1}$) of the residual oxidation-reduction substance may be obtained. In other words, the current peak may be obtained from a reduction current.

In the current peak measurement step (or the current response measurement step), the current peak measurement unit may perform electrochemical measurement (at least sweeping at voltages in a predetermined range (from a negative-side potential to a positive-side potential)) about the measurement solution containing the residual oxidation-reduction substance, which is not incorporated in the live bacteria, and may obtain the current peak ($I_{p1}$) of the residual oxidation-reduction substance. In other words, the current peak may be obtained from an oxidation current.

One kind or one or more kinds of current peaks may be obtained.

The first live bacteria quantifying method may include:

a data saving step of saving, in a storage medium, evaluation results obtained by the quantification evaluation step (for example, the number of live bacteria, the current peak difference ($\Delta I_p$), and the current peak ($I_{p1}$) of the residual oxidation-reduction substance); and

an output step of outputting the evaluation results obtained by the quantification evaluation step or the evaluation results saved in the storage medium to output means.

Examples of "output" may include displaying the evaluation results on display means, outputting the evaluation results to a printer and printing those, transmitting the evaluation results to an external device, and saving the evaluation results in the storage medium. It is sufficient that the external device or the storage medium is connected with a local area network or the Internet.

(Electrode Chip)

The electrode chip has the action electrode, the counter electrode, and the reference electrode or two electrodes of the action electrode and the reference electrode. The electrode chip is electrically connected with a power source. For example, the current peak can be obtained from a current waveform in a case where voltages in a predetermined range (from a positive-side potential to a negative-side potential or from a negative-side potential to a positive-side potential) are applied (sweeping) in accordance with a command from the current peak measurement unit.

The oxidation-reduction substance of an amount set in advance may be adhered to the electrode chip.

The electrode chip to which the oxidation-reduction substance is adhered may be placed in the measurement solution containing the live bacteria and a solvent, the oxidation-reduction substance may be dissolved, mixing may be performed for a predetermined period of time, and electrochemical measurement may be performed. After the oxidation-reduction substance adhered to the electrode chip is dissolved in the measurement solution, the live bacteria may be placed in the measurement solution and mixed for a predetermined period of time.

The electrode chip to which the oxidation-reduction substance is not adhered may be placed in the measurement solution containing the live bacteria, the oxidation-reduction substance, and the solvent, and electrochemical measurement may be performed.

The electrode chip may be an electrode of metal, carbon, electrically conductive glass, or the like or an electrode formed by metal plating or printing using electrically conductive ink.

A plurality of kinds of amounts of the adhered oxidation-reduction substance may be used.

The current peak measurement unit may include a single electrode which is formed with three electrodes of the action electrode, the counter electrode, and the reference electrode or with two electrodes of the action electrode and the reference electrode.

The current peak measurement unit may have:

a voltage application control unit that applies voltages in a predetermined range (from a positive potential to a negative potential or from a negative potential to a positive potential) according to the electrochemical measurement to the electrode chip or the action electrode and the reference electrode, which are formed as a single electrode; and

a current response measurement unit that obtains a current peak (peak height) of a current response which is measured by applying the voltages in the predetermined range (and a potential (peak potential) at the current peak).

The data accumulation unit may temporarily save the first and second collating data, may acquire the first and second collating data from an external device, and may be configured to be capable of updating the first and second collating data.

A first kit for quantifying live bacteria has

an oxidation-reduction substance which is cell-membrane-permeable for live bacteria as a measurement target, is oxidized or reduced in cells, and is deposited (insolubilized) in the cells. The first kit for quantifying live bacteria has the oxidation-reduction substance and a solvent. The oxidation-reduction substance and the solvent may separately and individually be packed in packages or vessels. The vessel for the solvent, in which the oxidation-reduction substance is placed, may be used as a measurement vessel.

A second kit for quantifying live bacteria includes
a measurement solution that contains an oxidation-reduction substance, which is cell-membrane-permeable for live bacteria as a measurement target, is oxidized or reduced in cells, and is deposited (insolubilized) in the cells, and a solvent. The measurement solution may be packed in a vessel or a package. The vessel or the package may be used as a measurement vessel.

Each of the first and second kits for quantifying live bacteria is used for measurement by the above quantifying device and/or quantifying method.

Each of the first and second kits for quantifying live bacteria may include a plurality of packages among which an amount of the oxidation-reduction substance is different.

Each of the first and second kits for quantifying live bacteria may include a plurality of packages among which a kind of the oxidation-reduction substance is different.

Each of the first and second kits for quantifying live bacteria may include a plurality of measurement vessels among which a concentration of the oxidation-reduction substance is different.

Each of the first and second kits for quantifying live bacteria may include a plurality of measurement vessels among which a kind of the oxidation-reduction substance is different.

(Live Bacteria)

The live bacteria are live cells, and examples may include Escherichia coli (E. coli), Salmonella enterica (S. enterica), Pseudomonas aeruginosa (P. aeruginosa), Staphylococcus aureus (S. aureus), and so forth.

(Oxidation-Reduction Substance)

The oxidation-reduction substance is a substance which is cell-membrane-permeable, is oxidized or reduced in cells, and is deposited in the cells. As the oxidation-reduction substance, artificial dyes can be raised. Examples of the artificial dyes may include tetrazolium salts. Examples of tetrazolium salts may include MTT (3-(4,5-di-methylthiazol-2-yl)-2,5-diphenyltetrazolium bromide), CTC (5-cyano-2,3-ditolyl tetrazolium chloride), INT (2-(4-iodophenyl)-3-(4-nitrophenyl)-5-phenyl-2H-tetrazolium chloride), XTT (2,3-bis-(2-methoxy-4-nitro-5-sulfophenyl)-2H-tetrazolium-5-carboxanilide), NBT (nitro blue tetrazolium), WST-8 (2-(2-methoxy-4-nitrophenyl)-3-(4-nitrophenyl)-5-(2,4-disulfophenyl)-2H-tetrazolium), 2,3,5-triphenyltetrazolium chloride, tetranitro blue tetrazolium, MTS (3-(4,5-dimethylthiazol-2-yl)-5-(3-carboxymethoxyphenyl)-2-(4-sulfophenyl)-2H-tetrazolium), m-tolyltetrazolium red, m-tolyltetrazolium red, 2,3-diphenyl-5-ethyltetrazolium chloride, 2,3-diphenyl-5-carboxytetrazolium chloride, 2,3,5-triphenyltetrazolium iodide, o-tolyltetrazolium red, tetrazolium violet, p-tolyltetrazolium red, 2,3,5-triphenyltetrazolium bromide, and so forth.

(Oxidation-Reduction Substance)

Examples of the solvent of the measurement solution may include tap water, pure water, purified water, and so forth. Those solvents may be subjected to a sterilization treatment (for example, 20 minutes at 121°C).

A computer program of another aspect of the disclosure
conducts each of the steps of the above live bacteria quantifying method when the computer program is executed by at least one processor.

A storage medium storing the computer program of another aspect of the disclosure
conducts each of the steps of the above live bacteria quantifying method when the computer program is executed by at least one processor.

An information processing device of another aspect of the disclosure includes:

at least one processor; and
a memory (which may be the above storage medium) in which a computer program to be executed by the processor is stored, and
each of the steps of the above live bacteria quantifying method is conducted when the computer program is executed by the at least one processor.

The information processing device is not particularly restricted, examples thereof may include a smartphone, a tablet, a smartwatch, a wearable computer, a personal computer, a local server, a cloud server, and so forth, and a single combination or a plurality of combinations of information processing devices may be configured to be connectable by wired and/or wireless communication means.

The live bacteria quantifying device may be configured to have a dedicated circuit, firmware, a processor, a memory storing a processing program, a display, a bus, an input-output interface, a communication device, and so forth, for example.

[Work and Effects]

In the present invention, the following work and effects are provided.

(1) Electrochemical evaluation of bacteria activity can simply be performed.
(2) A residual oxidation-reduction substance in a measurement solution is electrochemically quantified, a culture process for a long time becomes unnecessary, and rapid count of the number of live bacteria thereby becomes possible.
(3) Applications are possible to sanitary and quality management in medical, pharmaceutical, and food fields, environmental assessment (biofilm), predisease, and fields of prevention of specified diseases (intestinal flora).

Brief Description of Drawings

[Figure 1] Figure 1 is a function block diagram for explaining one example of functions of a live bacteria quantifying device.
[Figure 2A] Figure 2A is a diagram illustrating one example of collating data.
[Figure 2B] Figure 2B is a diagram illustrating one example of collating data.

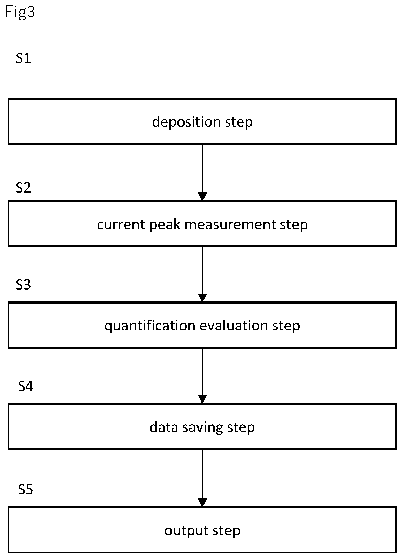

[Figure 3] Figure 3 is a flowchart of a live bacteria quantifying method.
[Figure 4A] Figure 4A is a diagram illustrating current response curves, an initial MTT current peak, and a residual MTT current peak in a practical example 1.
[Figure 4B] Figure 4B illustrates diagrams illustrating current peak differences corresponding to a live bacteria concentration (Escherichia coli) in the practical example 1.
[Figure 5A] Figure 5A is a diagram illustrating peak intensities in a comparative example.
[Figure 5B] Figure 5B illustrates diagrams illustrating peak intensities corresponding to the live bacteria concentration (Escherichia coli) in the comparative example.
[Figure 6A] Figure 6A is a diagram illustrating current response curves and four current peaks for Escherichia coli.
[Figure 6B] Figure 6B is a diagram illustrating changes in the four current peak differences which correspond to a stirring time for Escherichia coli.
[Figure 7A] Figure 7A is a diagram illustrating current response curves and four current peaks in another example (Escherichia coli).
[Figure 7B] Figure 7B is a diagram illustrating changes in the four current peak differences which correspond to the stirring time in the other example (Escherichia coli).
[Figure 8A] Figure 8A is a diagram illustrating current response curves and four current peaks in another example (Escherichia coli).
[Figure 8B] Figure 8B is a diagram illustrating changes in the four current peak differences which correspond to the stirring time in the other example (Escherichia coli).
[Figure 9A] Figure 9A is a diagram illustrating current response curves and four current peaks in another example (Salmonella enterica).
[Figure 9B] Figure 9B is a diagram illustrating changes in the four current peak differences which correspond to the stirring time in the other example (Salmonella enterica).
[Figure 10A] Figure 10A is a diagram illustrating current response curves and four current peaks in another example (Staphylococcus aureus).
[Figure 10B] Figure 10B is a diagram illustrating changes in the four current peak differences which correspond to the stirring time in the other example (Staphylococcus aureus).
[Figure 11A] Figure 11A is a diagram illustrating current response curves and four current peaks in another example (Pseudomonas aeruginosa).
[Figure 11B] Figure 11B is a diagram illustrating changes in the four current peak differences which correspond to the stirring time in the other example (Pseudomonas aeruginosa).
[Figure 12A] Figure 12A is a diagram illustrating current response curves and four current peaks in another example (four kinds of bacteria).

[Figure 12B] Figure 12B is a diagram illustrating changes in the four current peak differences which correspond to the stirring time in the other example (four kinds of bacteria).

[Figure 13A] Figure 13A is a diagram illustrating current response curves and one current peak in another example (NBT).

[Figure 13B] Figure 13B is a diagram illustrating a change in one current peak difference which corresponds to the stirring time in the other example (NBT).

[Figure 14] Figure 14 is a diagram illustrating a calibration curve which is obtained by plotting $\Delta I_p$ with respect to each concentration of Escherichia coli.

[Figure 15] Figure 15 is a diagram illustrating $\Delta I_p$ for one cell in each bacterium.

Description of Embodiment

(First Embodiment)

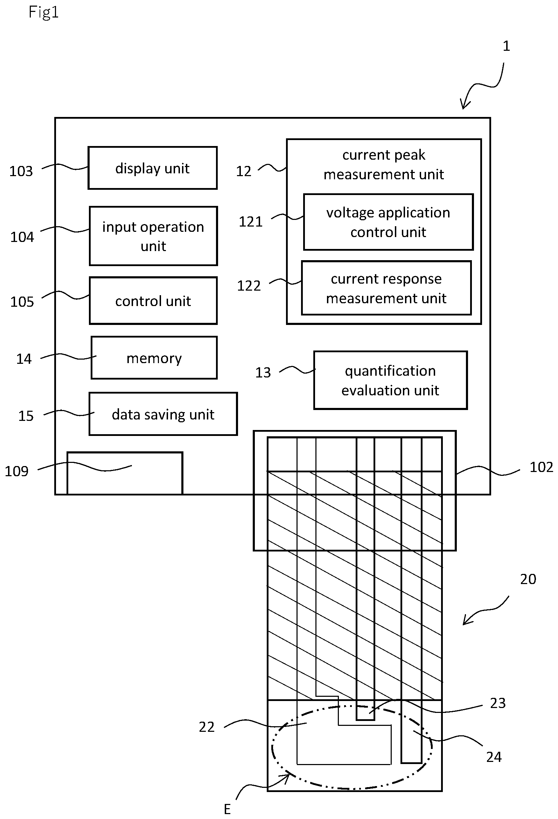

A live bacteria quantifying device 1 will be described with reference to Figure 1. The live bacteria quantifying device 1 includes a data accumulation unit 11, a current peak measurement unit 12, a quantification evaluation unit 13, a memory 14, a data saving unit 15, an electrode chip connection unit 102, a display unit 103, an input operation unit 104, a control unit 105, and a communication unit 109.

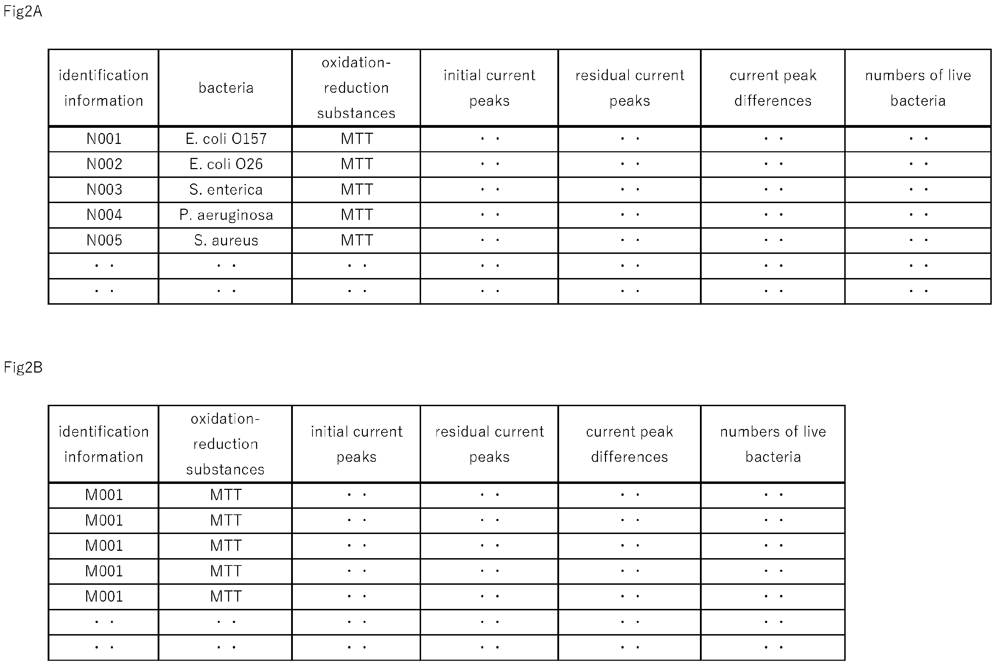

The data accumulation unit 11 is configured to have a storage medium and accumulates collating data including current peak differences ($\Delta I_p$) which are set in advance and the numbers of live bacteria which correspond to the current peak differences ($\Delta I_p$). Figure 2A illustrates one example of first collating data. The first collating data include identification information, bacteria, oxidation-reduction substances, initial current peaks, residual current peaks, current peak differences, and the numbers of live bacteria. The number of live bacteria in the first collating data indicates the number of designated bacteria. Figure 2B illustrates one example of second collating data. The second collating data include identification information, oxidation-reduction substances, initial current peaks, residual current peaks, current peak differences, and the numbers of live bacteria. The number of live bacteria in the second collating data indicates the number of all kinds of bacteria.

The current peak measurement unit 12 performs electrochemical measurement (sweeping from a predetermined positive potential to negative potential or from a predetermined negative potential to positive potential) about a measurement solution containing a residual oxidation-reduction substance, which is not incorporated in the live bacteria, and thereby obtains at least one current peak ($I_{p1}$) of the residual oxidation-reduction substance. The current peak measurement unit 12 has a voltage application control unit 121 which applies voltages in a predetermined range according to the electrochemical measurement to an action electrode 22 and a reference electrode 23 of the electrode chip 20 and a current response measurement unit 122 which obtains a current peak (peak height) of a current response which is measured by applying the voltages in the predetermined range (and a potential (peak potential) at the current peak).

Examples of the electrochemical measurement may include measurement methods using electrochemical techniques such as differential pulse voltammetry, normal pulse voltammetry, linear sweep voltammetry, stripping voltammetry, cyclic voltammetry, potentiometry, and amperometry.

The voltage application control unit 121 applies a predetermined constant voltage between the action electrode 22 and a counter electrode 24 of the electrode chip 20. The current response measurement unit 122 measures a current value which corresponds to the constant voltage applied between the action electrode 22 and the counter electrode 24. When the current value is obtained at the action electrode 22, the voltage application control unit 121 applies voltages in a predetermined range which repeat an equilibrium and sweep between the action electrode 22 and the reference electrode 23 in accordance with the electrochemical measurement. A current response measurement unit 121 measures the current value at the action electrode 22. The current response measurement unit 121 detects a measured current peak. A potential at the detected current peak will be referred to as a peak potential. The current response measurement unit 121 performs baseline fitting for measurement data about current responses, thereby obtains a baseline, and detects a peak height and a BG value with respect to the baseline as a reference. Further, the current response measurement unit 122 may calculate the peak height of the measured current peak or an area of a current peak curve.

The quantification evaluation unit 13 obtains a current peak difference ($\Delta I_p$) between a current peak ($I_{p0}$) of an initial oxidation-reduction substance, which is measured in advance, and the current peak ($I_{p1}$) of the residual oxidation-reduction substance, which is measured by the current peak measurement unit 12, performs collation with first or second collation data by the current peak difference ($\Delta I_p$), and thereby performs quantification evaluation of the live bacteria.

The data saving unit 15 saves evaluation results, which are obtained by the quantification evaluation unit 13, in the memory 14. The evaluation results are the number of live bacteria, the current peak difference ($\Delta I_p$), and the current peak ($I_{p1}$) of the residual oxidation-reduction substance, for example.

The display unit 103 displays the evaluation results obtained by the quantification evaluation unit 13 or the evaluation results saved in the memory 14 on a monitor (not illustrated).

The input operation unit 104 has an input interface, and an operator inputs necessary operation data and a necessary instruction.

The control unit 105 has a function of integrating and executing an operation process of the live bacteria quantifying device 1, processing of input and output data, and control of each function unit.

The communication unit 109 has wireless or wired communication means and transmits the evaluation results obtained by the quantification evaluation unit 13 or the evaluation results saved in the memory 14 to an external device. Further, the communication unit 109 receives the collating data from the external device. The control unit 105 sends the collating data to the data accumulation unit 11 via a bus in the device.

The electrode chip connection unit 102 is a structure on which the electrode chip 20 is electrically mounted.

The electrode chip 20 has three electrodes of the action electrode 22, the reference electrode 23, and the counter electrode 24. A suspension of the measurement solution is dropped to a sample dropping region E of the three electrodes, voltages in the predetermined range are applied, and the current response is thereby measured. The electrode chip 20 may be a disposable chip and may be reused after washing.

The current peak measurement unit 12, the voltage application control unit 121, the current response measurement unit 122, the quantification evaluation unit 13, the data saving unit 15, the control unit 105, the display unit 103, and the input operation unit 104 may be configured with a dedicated circuit, firmware, a computer program, hardware (such as a processor and a memory), and so forth.

(Live Bacteria Quantifying Method)

A live bacteria quantifying method includes the following process steps.

In a deposition step (S1), live bacteria are placed in a measurement vessel that contains an oxidation-reduction substance, which is cell-membrane-permeable for the live bacteria as a measurement target, is oxidized or reduced in cells, and is deposited (or insolubilized) in the cells, and are mixed for a predetermined period of time (for example, 20, 30, or 40 minutes). The oxidation-reduction substance in the measurement solution permeates cell membranes of the live bacteria and is diffused in the cells. The oxidation-reduction substance, for example, MTT is reduced to MTT formazan by electron carriers in the live bacteria such as NADH (nicotinamide adenine dinucleotide), NADPH (nicotinamide adenine dinucleotide phosphate), coenzymes, menaquinones, and ubiquinones. Because the MTT formazan is insoluble, the MTT formazan is deposited in the cells but is not again dissolved into the measurement solution.

In a current peak measurement step (S2), after the deposition step, the electrochemical measurement (sweeping at voltages in a predetermined range (from a positive-side potential to a negative-side potential or from a negative-side potential to a positive-side potential)) is performed for the measurement solution (which contains the live bacteria and the residual oxidation-reduction substance and is in a suspended state), and at least one current peak ($I_{p1}$) of the residual oxidation-reduction substance is thereby obtained.

The current peak measurement step may include a voltage application step of applying voltages in a predetermined range (from a positive-side potential to a negative-side potential or from a negative-side potential to a positive-side potential) according to the cyclic voltammetry measurement to the electrode chip or the action electrode and the reference electrode, which are formed as a single electrode, and a current response measurement step of obtaining the current peak (peak height) of the current response which is measured by applying the voltages in the predetermined range (and the potential (peak potential) at the current peak).

Examples of means for performing electrochemical detection, the means being used in the current peak measurement step, may include devices which measure current, voltage, electrical resistance, and impedance and methods which use electrochemical techniques such as differential pulse voltammetry, normal pulse voltammetry, linear sweep voltammetry, stripping voltammetry, cyclic voltammetry, potentiometry, and amperometry, which are controlled by software installed in a device.

In a quantification evaluation step (S3), the current peak difference ($\Delta I_p$) between the current peak ($I_{p0}$) of the initial oxidation-reduction substance, which is measured in advance, and the current peak ($I_{p1}$) of the residual oxidation-reduction substance, which is measured in the current peak measurement step, is obtained, and quantification evaluation of the live bacteria is performed based on the current peak difference ($\Delta I_p$). The oxidation-reduction substance in the measurement solution, for example, MTT decreases in accordance with an amount incorporated in the bacteria, the oxidation-reduction substance remaining in the measurement solution decreases as the number of bacteria increases, and the number of live bacteria can thereby be evaluated.

The live bacteria quantifying method may further include the following steps.

In a data saving step (S4), evaluation results obtained by the quantification evaluation step (for example, the number of live bacteria, the current peak difference ($\Delta I_p$), and the current peak ($I_{p1}$) of the residual oxidation-reduction substance) are saved in the memory.

In an output step (S5), the evaluation results obtained by the quantification evaluation step or the evaluation results saved in the memory are output to output means.

Each of the above steps may be realized by using the live bacteria quantifying device 1 of the first embodiment or may be realized by a common electrochemical measurement device or information processing device.

[Kit for Quantifying Live Bacteria]

A measurement solution that contains the oxidation-reduction substance, which is cell-membrane-permeable for the live bacteria as the measurement target, is oxidized or reduced in the cells, and is deposited (or insolubilized) in the cells, and a solvent is packed in a measurement vessel. After the live bacteria are placed in the measurement vessel and are mixed, a resulting suspension can be measured.

When the kit packs only the oxidation-reduction substance, the live bacteria and water (solvent) are placed in the vessel, the oxidation-reduction substance is placed and mixed, and a resulting suspension can be measured.

It is preferable that a packing article be a vessel which tightly seals a content and in which its physical properties are not degraded, and examples may include plastic-made bottle vessels, film-made packing vessels, glass vessels, and so forth.

(Practical Examples)

Practical examples were conducted under the following conditions.

Oxidation-reduction substances: MTT (operation procedures 1 to 3) and NBT (operation procedure 4)
Initial concentration of MTT and NBT in the measurement solution: 0.10 mM
Solvent of the measurement solution: pure water subjected to a sterilization treatment
Live bacteria: Escherichia coli K12 (operation procedures 1 to 3), Salmonella enterica, Staphylococcus aureus, and Pseudomonas aeruginosa (operation procedure 3)
Live bacteria concentrations in the measurement solution: $1 \times 10^3$, $1 \times 10^4$, $1 \times 10^5$, $1 \times 10^6$, and $1 \times 10^7$ [CFUmL$^{-1}$]
Deposition times: 37°C, 1 to 10, 20, 30, 40, 50, and 60 minutes (contacting-mixing times)
Electrochemical measurement: cyclic voltammetry measurement device
Electrodes: a glassy carbon electrode as the action electrode, a platinum coil electrode as the counter electrode, and a silver-silver chloride electrode as the reference electrode
Sweep voltages: voltages of +0.2 V to -0.4 V are applied.
: sweeping from +0.6 V to -0.6 V is performed, and sweeping from -0.6 V to +0.6 V is performed.

<Operation Procedure 1: Practical Example 1>

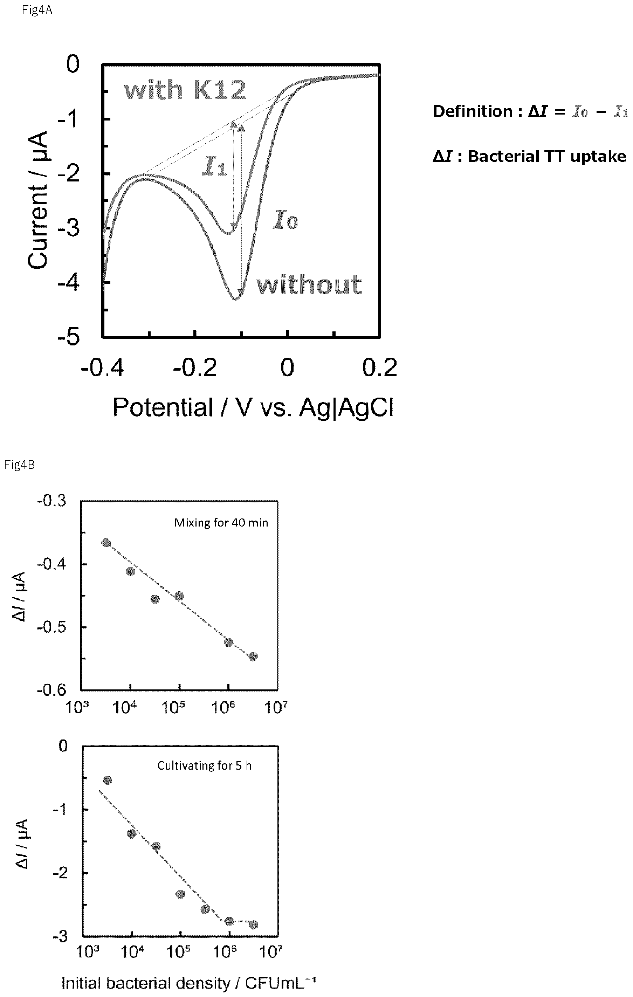

(1) Escherichia coli K12 is placed in a vessel in which the measurement solution containing MTT is placed.
(2) Mixing is performed for 40 minutes. MTT permeates the cell membranes of the bacteria, is reduced in the cells, becomes the MTT formazan, and is deposited.
(3) A suspension is taken out from the measurement vessel and is dropped to the electrode chip.
(4) The cyclic voltammetry measurement is performed. Voltages of +0.2 V to -0.4 V are applied, and a resulting current response is measured.
(5) A residual MTT current peak ($I_{p1}$) [$\mu$A] is obtained from the current response. The current peak can be observed around -0.12 V (see Figure 4A).
(6) The current peak difference ($\Delta I_p$) between an initial MTT current peak ($I_{p0}$) and the residual MTT current peak ($I_{p1}$) is obtained.

$$\Delta I_p = I_{p0} - I_{p1}$$

(7) Quantification evaluation of the live bacteria is performed based on the current peak difference ($\Delta I_p$). An amount of MTT which is incorporated by the live bacteria and deposited is obtained from the current peak difference ($\Delta I_p$). The number of live bacteria is obtained from the obtained amount of MTT. An amount of MTT which can be incorporated in the live bacteria is set in advance (experimentally obtained).

It was observed that the current peak difference ($\Delta I_p$) decreased in proportion to an increase in a concentration of Escherichia coli from $10^3$ to $10^7$ [CFUmL$^{-1}$] (see Figure 4B). In a comparative example which will be described later, quantification could not be performed unless the concentration was of $10^4$.

<Operation Procedure 2: Practical Example 2>

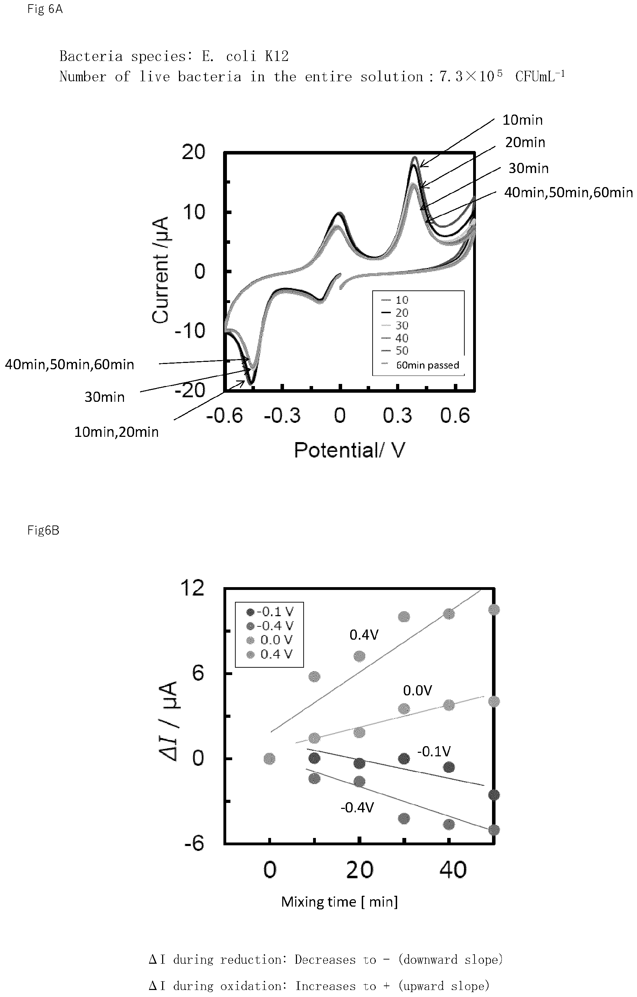

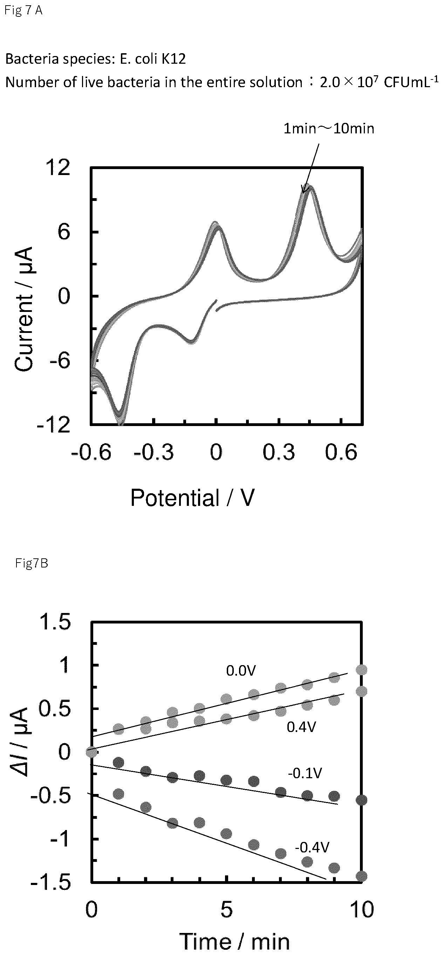

(1) Escherichia coli K12 (the number of live bacteria in the whole measurement solution in Figure 6A is $7.3 \times 10^5$ CFUmL$^{-1}$, and the number of live bacteria in the whole measurement solution in Figure 7A is $2.0 \times 10^7$ CFUmL$^{-1}$) is placed in the vessel in which the measurement solution containing MTT is placed.
(2) Mixing is performed for 1 to 10, 20, 30, 40, 50, and 60 minutes. MTT permeates the cell membranes of the bacteria, is reduced in the cells, becomes the MTT formazan, and is deposited.
(3) After mixing is performed for 1 to 10, 20, 30, 40, 50, and 60 minutes, respective suspensions are taken out from the measurement vessel and are dropped to the electrode chip.
(4) The cyclic voltammetry measurement is performed. Sweeping from +0.6 V to -0.6 V is performed, sweeping from -0.6 V to +0.6 V is performed, and resulting current responses are measured.
(5) The residual MTT current peak ($I_{p1}$) [$\mu$A] is obtained from the current response. The current peaks can be observed around -0.1 V and around -0.4 V in the sweeping in a reduced condition, and the current peaks can be observed around 0 V and around +0.4 V in the sweeping in an oxidized condition (see Figure 6A and Figure 7A).
(6) The current peak difference ($\Delta I_p$) between the initial MTT current peak ($I_{p0}$) and the residual MTT current peak ($I_{p1}$) is obtained at each of the current peaks.

$$\Delta I_p = I_{p0} - I_{p1}$$

Figure 6B illustrates the respective current peak differences ($\Delta I_p$) at the peaks at -0.1 V, -0.4 V, 0 V, and +0.4 V at stirring times of 10, 20, 30, 40, and 50 minutes.
Figure 7B illustrates the respective current peak differences ($\Delta I_p$) at the peaks at -0.1 V, -0.4 V, 0 V, and +0.4 V at stirring times of 1 to 10 minutes.

(7) Quantification evaluation of the live bacteria is performed based on the current peak differences ($\Delta I_p$). The amounts of MTT which is incorporated by the live bacteria and deposited are obtained from the current peak differences ($\Delta I_p$). The numbers of live bacteria are obtained from the obtained amounts of MTT. The amounts of MTT which can be incorporated in the live bacteria are set in advance (experimentally obtained).

Based on results of the operation procedure 2, it was observed that the number of live bacteria could be quantified from the current peak difference ($\Delta I_p$) with a mixing time of 1 minute or longer, and it was indicated that quantification could more precisely be performed with a mixing time of 10 minutes or longer. Further, it was observed that precision of quantification was enhanced in proportion to the concentration of the live bacteria.

<Operation Procedure 3: Practical Example 3>

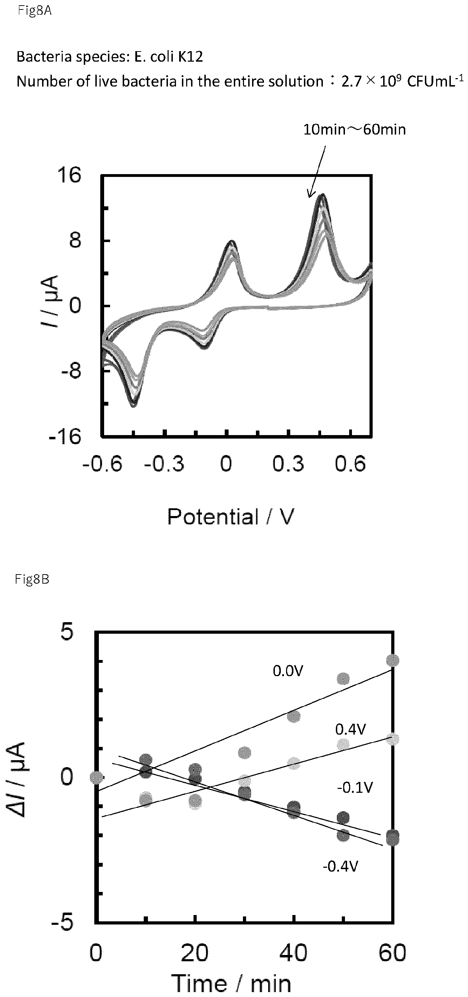

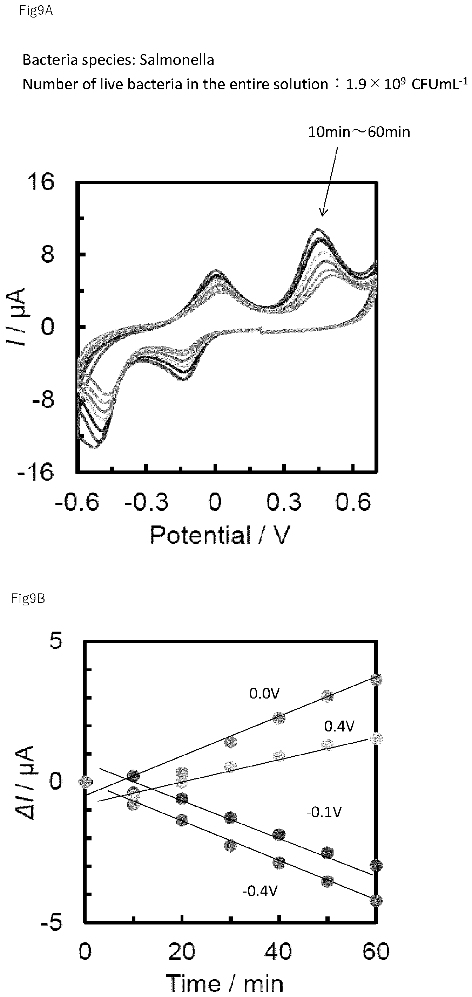

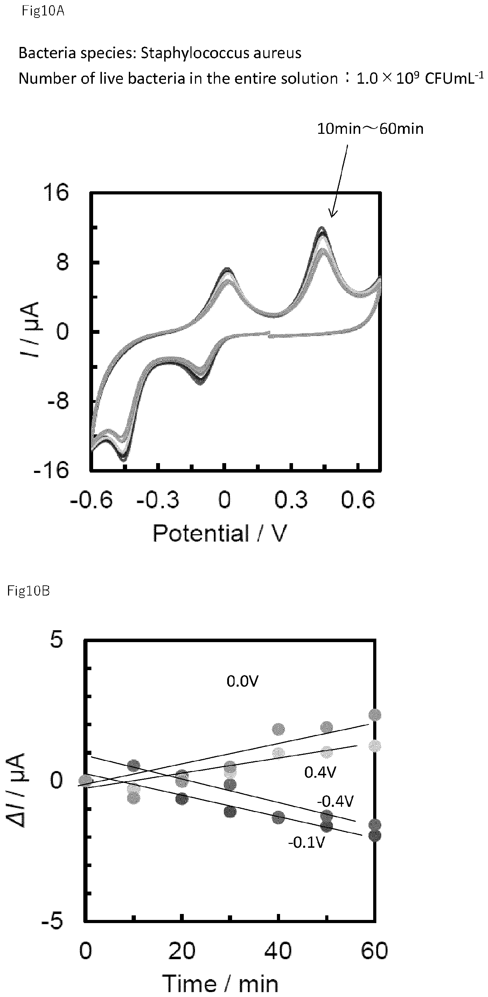

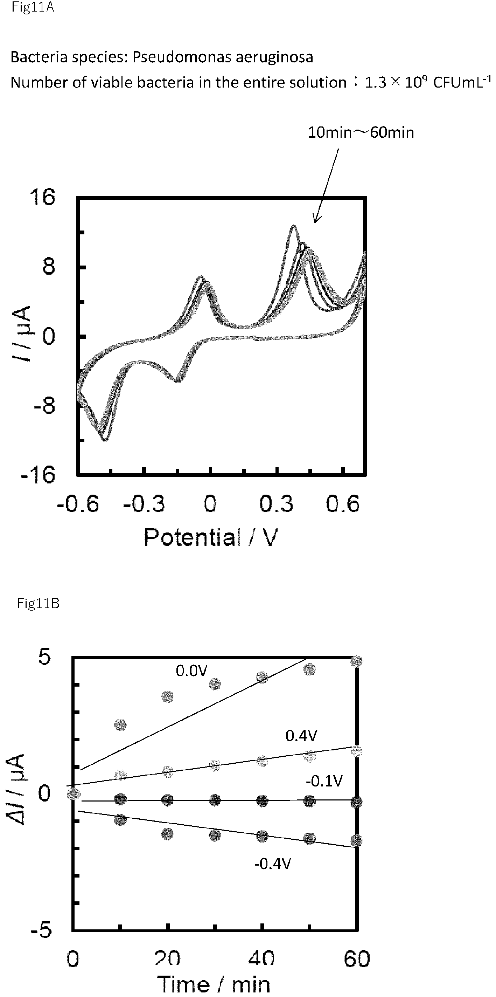

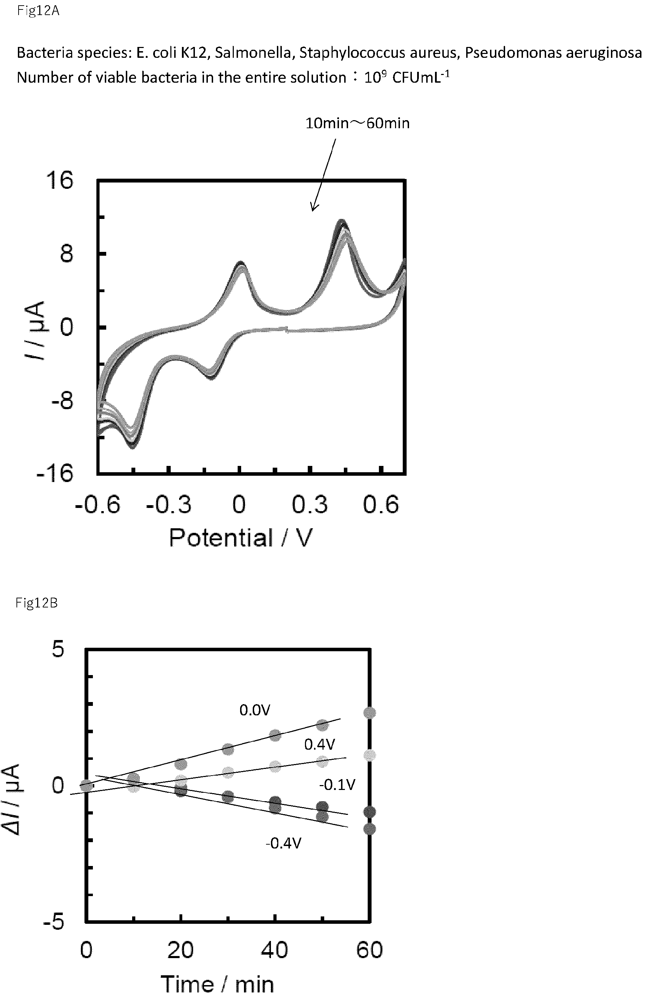

(1) Escherichia coli K12 (the number of live bacteria in the whole measurement solution in Figure 8A is $2.7 \times 10^9$ CFUmL$^{-1}$) is placed in a vessel 1 in which the measurement solution containing MTT is placed, Salmonella enterica (the number of live bacteria in the whole measurement solution in Figure 9A is $1.9 \times 10^9$ CFUmL$^{-1}$) is placed in a vessel 2, Staphylococcus aureus (the number of live bacteria in the whole measurement solution in Figure 10A is $1.0 \times 10^9$ CFUmL$^{-1}$) is placed in a vessel 3, Pseudomonas aeruginosa (the number of live bacteria in the whole measurement solution in Figure 11A is $1.3 \times 10^9$ CFUmL$^{-1}$) is placed in a vessel 4, and MTT is deposited in each of the bacteria.
Further, Escherichia coli K12, Salmonella enterica, Staphylococcus aureus, and Pseudomonas aeruginosa (the number of all of the live bacteria in the whole measurement solution in Figure 12A is $10^9$ CFUmL$^{-1}$) are placed in a vessel 5 in which the measurement solution containing MTT is placed, and MTT is deposited.
(2) Mixing is performed for 1 to 10, 20, 30, 40, 50, and 60 minutes. MTT permeates the cell membranes of the bacteria, is reduced in the cells, becomes the MTT formazan, and is deposited.
(3) After mixing is performed for 1 to 10, 20, 30, 40, 50, and 60 minutes, respective suspensions are taken out from the measurement vessels 1 to 5 and are dropped to the electrode chip.
(4) The cyclic voltammetry measurement is performed. Sweeping from +0.6 V to -0.6 V is performed, sweeping from -0.6 V to +0.6 V is performed, and resulting current responses are measured.
(5) The residual MTT current peak ($I_{p1}$) [$\mu$A] is obtained from the current response. The current peaks can be observed around -0.1 V and around -0.4 V in the sweeping in the reduced condition, and the current peaks can be observed around 0 V and around +0.4 V in the sweeping in the oxidized condition (see Figure 8A to Figure 12A).

(6) The current peak difference ($\Delta I_p$) between the initial MTT current peak ($I_{p0}$) and the residual MTT current peak ($I_{p1}$) is obtained at each of the current peaks.

$$\Delta I_p = I_{p0} - I_{p1}$$

Figure 8B to Figure 12B illustrate the respective current peak differences ($\Delta I_p$) at the peaks at -0.1 V, - 0.4 V, 0 V, and +0.4 V at stirring times of 10, 20, 30, 40, and 50 minutes.

(7) Quantification evaluation of the live bacteria is performed based on the current peak differences ($\Delta I_p$). The amounts of MTT which is incorporated by the live bacteria and deposited are obtained from the current peak differences ($\Delta I_p$). The numbers of live bacteria are obtained from the obtained amounts of MTT. The amounts of MTT which can be incorporated in the live bacteria are set in advance (experimentally obtained).

Results of the operation procedure 3 indicated that it was possible to observe that each of the numbers of live bacteria could be quantified from the current peak difference ($\Delta I_p$) with a mixing time of 1 minute or longer. Further, it was observed that a plurality of kinds of live bacteria could collectively be quantified at the same time.

<Operation Procedure 4: Practical Example 4>

(1) Escherichia coli K12 (the number of live bacteria in the whole measurement solution is approximately $10^8$ to $10^9$ CFUmL$^{-1}$) is placed in a vessel in which the measurement solution containing NBT is placed, and NBT is deposited in the bacteria.

(2) Mixing is performed for 10, 20, 30, 40, 50, and 60 minutes. NBT permeates the cell membranes of the bacteria, is reduced in the cells, becomes an NBT formazan, and is deposited.

(3) After mixing is performed for 10, 20, 30, 40, 50, and 60 minutes, respective suspensions are taken out from the measurement vessel and are dropped to the electrode chip.

(4) The cyclic voltammetry measurement is performed. Sweeping from +0.2 V to -0.6 V is performed, sweeping from -0.6 V to +0.6 V is performed, and resulting current responses are measured.

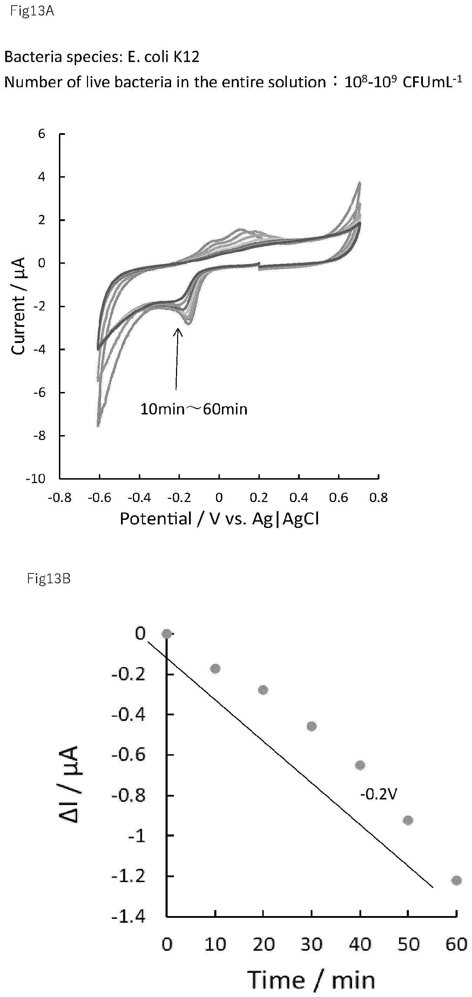

(5) A residual NBT current peak ($I_{p1}$) [$\mu$A] is obtained from the current response. One current peak can be observed around -0.2 V in sweeping in a reduced condition (see Figure 13A).

(6) The current peak difference ($\Delta I_p$) between an initial NBT current peak ($I_{p0}$) and the residual NBT current peak ($I_{p1}$) is obtained at one current peak.

$$\Delta I_p = I_{p0} - I_{p1}$$

Figure 13B illustrates the current peak differences ($\Delta I_p$) at the peak at -0.2 V at stirring times of 10, 20, 30, 40, and 50 minutes.

(7) Quantification evaluation of the live bacteria is performed based on the current peak differences ($\Delta I_p$). The amounts of NBT which is incorporated by the live bacteria and deposited are obtained from the current peak differences ($\Delta I_p$). The numbers of live bacteria are obtained from the obtained amounts of NBT. The amounts of NBT which can be incorporated in the live bacteria are set in advance (experimentally obtained).

(Comparative Example)

As a comparative example, evaluation by spectroscopic measurement (method in related art) was performed.

Artificial dye: MTT
Initial concentration of MTT in the measurement solution: 0.10 mM
Solvent of the measurement solution: pure water subjected to a sterilization treatment
Live bacteria: Escherichia coli K12
Live bacteria concentrations in the measurement solution: $1 \times 10^3$, $1 \times 10^4$, $1 \times 10^5$, $1 \times 10^6$, and $1 \times 10^7$ [CFUmL$^{-1}$]
Culturing time: 37°C, 40 minutes and 5 hours
Spectroscopic optical measurement: absorbance meter

<Operation Procedure 1: Comparative Example 1>

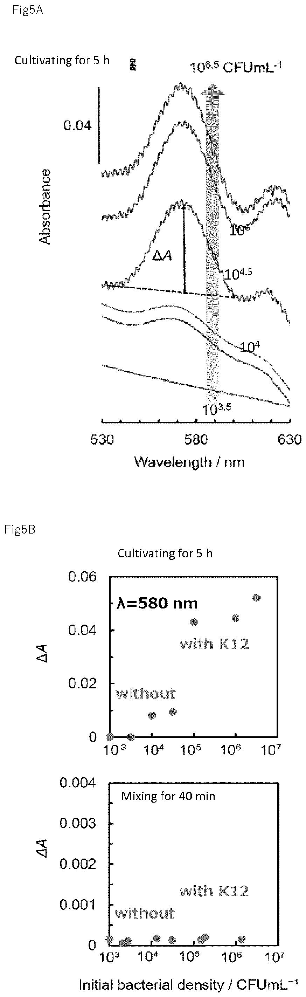

(1) Escherichia coli K12 is placed in a vessel in which the measurement solution containing MTT is placed.
(2) Culture is performed.
(3) An absorbance spectrum of the measurement solution is measured by a spectrophotometer. A peak intensity is exhibited in a region of a wavelength of 580 nm (see Figure 5A).
(4) From the peak intensity at a wavelength of 580 nm, a concentration of MTT formazan deposited in the live bacteria is obtained. In Figure 5A, $\Delta A$ indicates absorbance of the MTT formazan.

In 5-hour culture, the peak intensity could be observed from Escherichia coli concentrations of $10^4$ to $10^7$ [$CFUmL^{-1}$], but quantification was not possible at a concentration of $10^3$ due to a low peak intensity (see Figures 5A and 5B).

As for 40-minute stirring, the live bacteria could not be quantified because the peak intensity was at the detection limit or lower and could not be observed.

(Practical Example Indicating Correlation with Official Method)

Standards and criteria of official methods are defined for target foods, those are basically indicated by announced methods, and further, individual testing methods which correspond to cases where a problem in food sanitation has occurred are indicated by notified methods. Only methods indicated by announcements and notifications are understood as the official methods. A laboratory conducting the official method not only has to follow a protocol (such as kinds of culture media and testing procedures) of the testing method but also has to certify that the laboratory, which includes specifications of used apparatuses, skills of persons in charge, and so forth, has a system that is "capable of conducting an appropriate test". Internal quality control (internal accuracy control) of the laboratory is a mandatory requirement, and acquisition of the certification of ISO 17025 is required. A Petrifilm inspection, which is one of standard inspection methods described in "Standard Methods of Analysis in Food Safety Regulation, Microbiological Section" supervised by the Ministry of Health, Labour and Welfare has widely been used as a simple method (rapid method) in the field because the Petrifilm inspection has a proper correlation with the official method. A 3M Petrifilm (TM) Aerobic Count Plate (AC Plate) is a testing method certified by AOAC and AFNOR as international third party organizations. As for the former, the FDA-BAM as AOAC-OMA (official methods of analysis) (certification numbers 986.33, 989.10, and 990.12) is used as a reference method, as for the latter, NF ISO 4833-1 (Oct 2013) as AFNOR (certification number 3M 01/01-09/89) is used as the reference method, and validity of those has been confirmed. Further, because the 3M Petrifilm (TM) Aerobic Count Plate (AC Plate) is a standard inspection method published in Standard Methods of Analysis in Food Safety Regulation in Japan and has a proper correlation with the official method, comparison between a simple method using the AC plate and the present invention was performed.

(Practical Example)

The practical example was conducted under the following conditions.

Oxidation-reduction substance: MTT
Initial concentration of MTT in the measurement solution: 0.10 mM
Solvent of the measurement solution: a phosphate buffer solution subjected to a sterilization treatment
Live bacteria: Escherichia coli K12
Live bacteria concentration in the measurement solution: $10^7$ [$CFUmL^{-1}$]
Deposition time: 37°C, 60 minutes (contacting-mixing time)
Electrochemical measurement: cyclic voltammetry measurement device
Electrodes: a glassy carbon electrode as the action electrode, a platinum coil electrode as the counter electrode, and a silver-silver chloride electrode as the reference electrode
Sweep voltages: sweeping from +0.2 V to -0.3 V is performed, and sweeping from -0.3 V to +0.7 V is performed.

Official method: the measurement solution, which contains MTT, of 1.0 mL was dropped to the 3M Petrifilm (TM) Aerobic Count Plate (AC Plate) and was cultured at 37°C for 24 hours. Dyed colonies which were formed on a sheet were counted.

<Operation Procedure 5: Practical Example 5>

(1) Escherichia coli K12 is individually placed in vessels in which the measurement solution containing MTT is placed, concentrations are adjusted to $4.2 \times 10^2$, $4.2 \times 10^3$, $4.2 \times 10^4$, $4.2 \times 10^5$, $4.2 \times 10^6$, and $4.2 \times 10^7$ [CFUmL$^{-1}$], and mixing is performed for 60 minutes. MTT permeates the cell membranes of the bacteria, is reduced in the cells, becomes the MTT formazan, and is deposited.

(2) The cyclic voltammetry measurement is performed. Voltages of +0.2 V to -0.3 V are applied, and a resulting current response is measured.

(3) The residual MTT current peak ($I_{p1}$) [μA] is obtained from the current response. The current peak can be observed around -0.12 V.

(4) The current peak difference ($\Delta I_p$) between the initial MTT current peak ($I_{p0}$) and the residual MTT current peak ($I_{p1}$) is obtained (see Table 1).

$$\Delta I_p \; = \; I_{p0} \; - \; I_{p1}$$

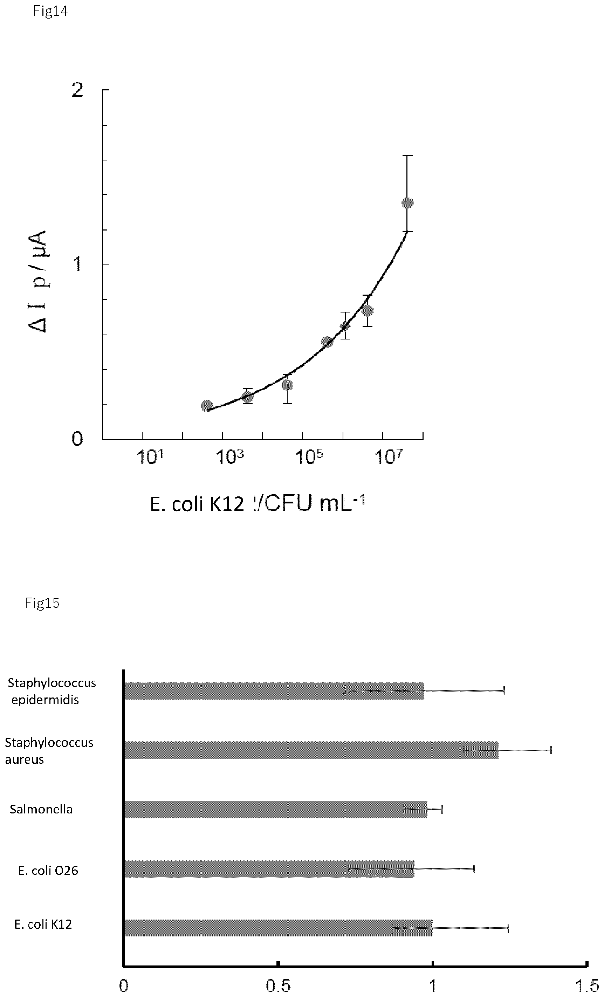

(5) By plotting $\Delta I_p$ with respect to each of the concentrations of Escherichia coli, a calibration curve is created (see Figure 14).

[Table 1]

| Standard Inspection Method (CFUmL $^1$) | $\Delta Ip(\mu A)$ |
|---|---|
| 420 | 0.19 |
| 4200 | 0.24 |
| 42000 | 0.31 |
| 420000 | 0.56 |
| 4200000 | 0.74 |
| 42000000 | 1.35 |

<Operation Procedure 6: Practical Example 6>

(1) Minced chicken meat of 100 g was kept still at 30°C for 50 hours, caused to decay, and dispersed in 100 mL of sterile water. The dispersion liquid of 50 μL is placed in a vessel in which the measurement solution containing MTT is placed and mixed for 60 minutes. MTT permeates the cell membranes of the bacteria, is reduced in the cells, becomes the MTT formazan, and is deposited.

(2) The cyclic voltammetry measurement is performed. Voltages of +0.2 V to -0.3 V are applied, and a resulting current response is measured. The residual MTT current peak ($I_{p1}$) [μA] is obtained from the current response. The current peak can be observed around -0.12 V.

(3) The current peak difference ($\Delta I_p$) between the initial MTT current peak ($I_{p0}$) and the residual MTT current peak ($I_{p1}$) is obtained, $\Delta I_p$ are plotted on the calibration curve created in <operation procedure 5>, and the number of live bacteria is calculated (see Table 2).

$$\Delta I_p \; = \; I_{p0} \; - \; I_{p1}$$

(4) The same measurement solution was dropped to the 3M Petrifilm (TM) Aerobic Count Plate (AC Plate), and the number of live bacteria was obtained by counting the colonies (see Table 1).

(5) The numbers of live bacteria obtained by the present method and the standard inspection method were $1.08 \pm 0.02 \times 10^6$ CFUmL$^{-1}$ and $1.17 \pm 0.03 \times 10^6$ CFUmL$^{-1}$, and an error between those was within 10%.

(6) Note that common live bacteria, lactic acid bacteria, psychrophilic bacteria, and fungi were contained in the measurement solution.

[Table 2]

| Standard Inspection Method (CFUmL$^{-1}$) | $\Delta$Ip($\mu$A) | Present Method (CFU/mL) |
|---|---|---|
| $1.17\pm0.03\times10^6$ | 0.649 | $1.08\pm0.02\times10^6$ |

<Operation Procedure 7: Practical Example 7>

(1) Escherichia coli K12, Escherichia coli O26, Salmonella enterica, Staphylococcus aureus, and Staphylococcus epidermidis are individually placed in vessels in which the measurement solution containing MTT is placed and are mixed for 60 minutes. MTT permeates the cell membranes of the bacteria, is reduced in the cells, becomes the MTT formazan, and is deposited.

(3) The cyclic voltammetry measurement is performed. Voltages of +0.2 V to -0.3 V are applied, and a resulting current response is measured.

(4) The residual MTT current peak ($I_{p1}$) [$\mu$A] is obtained from the current response. The current peak can be observed around -0.12 V.

(5) The current peak difference ($\Delta I_p$) between an initial MTT current peak ($I_{p0}$) and the residual MTT current peak ($I_{p1}$) is obtained.

$$\Delta I_p = I_{p0} - I_{p1}$$

(6) The current peak difference ($\Delta I_p$) of each of Escherichia coli K12, Escherichia coli O26, Salmonella enterica, Staphylococcus aureus, and Staphylococcus epidermidis is obtained (see Table 3).

(7) The same measurement solution was dropped to the 3M Petrifilm (TM) Aerobic Count Plate (AC Plate), and the numbers of live bacteria were obtained by counting the colonies (see Table 3).

(8) The current peak differences $\Delta I_p$ of Escherichia coli K12, Escherichia coli O26, Salmonella enterica, Staphylococcus aureus, and Staphylococcus epidermidis were divided by the respective numbers of live bacteria which are obtained by counting the colonies, and $\Delta I_p$ for one cell was thereby calculated (see Table 3). While Escherichia coli K12 is set as a reference (1.00), ratios of Escherichia coli O26, Salmonella enterica, Staphylococcus aureus, and Staphylococcus epidermidis are obtained. The ratios of Escherichia coli O26, Salmonella enterica, Staphylococcus aureus, and Staphylococcus epidermidis were 0.94 to 1.21 and were approximately the same as the ratio of Escherichia coli K12 (see Figure 15).

[Table 3]

| | $\Delta$Ip ($\mu$A) | Standard Inspection Method (CFU/mL) | $\Delta$Ip/cell ($\mu$A/cell) | $\Delta$Ip/cell ratio |
|---|---|---|---|---|
| Escherichia coli K12 | 1.43 | $6.9\times10^7$ | $2.1\times10^{-8}$ | 1.00 |
| Escherichia coli O26 | 2.45 | $1.3\times10^8$ | $1.9\times10^{-8}$ | 0.94 |
| Salmonella enterica | 0.63 | $3.1\times10^7$ | $2.0\times10^{-8}$ | 0.98 |
| Staphylococcus aureus | 1.82 | $7.2\times10^7$ | $2.5\times10^{-8}$ | 1.21 |
| Staphylococcus epidermidis | 0.52 | $2.6\times10^7$ | $2.0\times10^{-8}$ | 0.97 |

Reference Signs List

1       live bacteria quantifying device

12      current peak measurement unit

121     voltage application control unit

122     current response measurement unit

13     quantification evaluation unit

14     memory

15     data saving unit

103    display unit

104    input operation unit

105    control unit

109    communication unit

20     electrode chip

22     action electrode

23     reference electrode

24     counter electrode

**Claims**

1. A live bacteria quantifying method comprising:

   a deposition step in which live bacteria are placed in a measurement solution and are mixed for a predetermined period of time, the measurement solution containing an oxidation-reduction substance which is cell-membrane-permeable for the live bacteria as a measurement target, is oxidized or reduced in cells, and is deposited in the cells of the live bacteria, and the oxidation-reduction substance is caused to be incorporated and deposited in the cells of the live bacteria;
   a current peak measurement step in which after the deposition step, electrochemical measurement is performed for the measurement solution and a current peak of a residual oxidation-reduction substance remaining in the measurement solution is obtained; and
   a quantification evaluation step in which a current peak difference ($\Delta I_p$) between at least one current peak ($I_{p1}$) of the residual oxidation-reduction substance, the current peak ($I_{p1}$) being measured in the current peak measurement step, and a current peak ($I_{p0}$) of an initial oxidation-reduction substance, the current peak ($I_{p0}$) corresponding to the at least one current peak ($I_{p1}$) and being set in advance, is obtained and quantification evaluation of the live bacteria is performed based on the current peak difference ($\Delta I_p$).

2. A live bacteria quantifying device comprising:

   a data accumulation unit that accumulates collating data which are set in advance;
   a current peak measurement unit that performs electrochemical measurement about a measurement solution containing a residual oxidation-reduction substance, which is not incorporated in live bacteria, and that obtains at least one current peak ($I_{p1}$) of the residual oxidation-reduction substance; and
   a quantification evaluation unit that obtains a current peak difference obtaining a current peak difference ($\Delta I_p$) between the at least one current peak ($I_{p1}$) of the residual oxidation-reduction substance, the current peak ($I_{p1}$) being measured by the current peak measurement unit, and a current peak ($I_{p0}$) of an initial oxidation-reduction substance, the current peak ($I_{p0}$) corresponding to the at least one current peak ($I_{p1}$) and being set in advance, that performs collation with collation data of the data accumulation unit by the current peak difference ($\Delta I_p$), and that performs quantification evaluation of the live bacteria.

3. The live bacteria quantifying device according to claim 1, comprising
   an electrode chip to which the oxidation-reduction substance of an amount set in advance is adhered.

4. A kit for quantifying live bacteria, the kit being used for the live bacteria quantifying method according to claim 1 or being used for the live bacteria quantifying device according to claim 2 or 3, the kit comprising

an oxidation-reduction substance which is cell-membrane-permeable for the live bacteria as a measurement target, is oxidized or reduced in cells, and is deposited in the cells.

Fig1

display unit — 103

input operation unit — 104

control unit — 105

memory — 14

data saving unit — 15

109

current peak measurement unit — 12

voltage application control unit — 121

current response measurement unit — 122

quantification evaluation unit — 13

102

1

20

22

23

24

E

Fig2A

| identification information | bacteria | oxidation-reduction substances | initial current peaks | residual current peaks | current peak differences | numbers of live bacteria |
|---|---|---|---|---|---|---|
| N001 | E. coli O157 | MTT | · · | · · | · · | · · |
| N002 | E. coli O26 | MTT | · · | · · | · · | · · |
| N003 | S. enterica | MTT | · · | · · | · · | · · |
| N004 | P. aeruginosa | MTT | · · | · · | · · | · · |
| N005 | S. aureus | MTT | · · | · · | · · | · · |
| · · | · · | · · | · · | · · | · · | · · |
| · · | · · | · · | · · | · · | · · | · · |

Fig2B

| identification information | oxidation-reduction substances | initial current peaks | residual current peaks | current peak differences | numbers of live bacteria |
|---|---|---|---|---|---|
| M001 | MTT | · · | · · | · · | · · |
| M001 | MTT | · · | · · | · · | · · |
| M001 | MTT | · · | · · | · · | · · |
| M001 | MTT | · · | · · | · · | · · |
| M001 | MTT | · · | · · | · · | · · |
| · · | · · | · · | · · | · · | · · |
| · · | · · | · · | · · | · · | · · |

Fig3

S1

```
┌─────────────────────────────────────────────┐
│              deposition step                  │
└─────────────────────────────────────────────┘
```

S2

```
┌─────────────────────────────────────────────┐
│       current peak measurement step           │
└─────────────────────────────────────────────┘
```

S3

```
┌─────────────────────────────────────────────┐
│        quantification evaluation step         │
└─────────────────────────────────────────────┘
```

S4

```
┌─────────────────────────────────────────────┐
│              data saving step                 │
└─────────────────────────────────────────────┘
```

S5

```
┌─────────────────────────────────────────────┐
│                output step                    │
└─────────────────────────────────────────────┘
```

Fig4A

Definition : $\Delta I = I_0 - I_1$

$\Delta I$ : Bacterial TT uptake

Fig4B

Fig5A

Cultivating for 5 h

Fig5B

Fig 6A

Bacteria species: E. coli K12
Number of live bacteria in the entire solution : $7.3 \times 10^5$ CFUmL$^{-1}$

Fig6B

ΔI during reduction: Decreases to − (downward slope)

ΔI during oxidation: Increases to + (upward slope)

Fig 7 A

Bacteria species: E. coli K12
Number of live bacteria in the entire solution : 2.0 × 10^7 CFUmL^-1

1min〜10min

Fig7B

Fig8A

Bacteria species: E. coli K12
Number of live bacteria in the entire solution : $2.7 \times 10^9$ CFUmL$^{-1}$

Fig8B

Fig9A

Bacteria species: Salmonella
Number of live bacteria in the entire solution ： 1.9 × 10$^9$ CFUmL$^{-1}$

10min～60min

Fig9B

Fig10A

Bacteria species: Staphylococcus aureus
Number of live bacteria in the entire solution : $1.0 \times 10^9$ CFUmL$^{-1}$

10min〜60min

Fig10B

Fig11A

Bacteria species: Pseudomonas aeruginosa
Number of viable bacteria in the entire solution ： $1.3 \times 10^9$ CFUmL$^{-1}$

Fig11B

Fig12A

Bacteria species: E. coli K12, Salmonella, Staphylococcus aureus, Pseudomonas aeruginosa
Number of viable bacteria in the entire solution ： $10^9$ CFUmL$^{-1}$

Fig12B

Fig13A

Bacteria species: E. coli K12
Number of live bacteria in the entire solution : $10^8$-$10^9$ CFUmL$^{-1}$

Fig13B

Fig14

Fig15

## INTERNATIONAL SEARCH REPORT

| International application No. |
|---|
| **PCT/JP2023/016896** |

**A. CLASSIFICATION OF SUBJECT MATTER**

*C12N 1/20*(2006.01)i; *C12Q 1/06*(2006.01)i; *G01N 27/48*(2006.01)i; *C12M 1/34*(2006.01)i
FI: C12Q1/06; C12M1/34 B; G01N27/48 311; C12N1/20 A

According to International Patent Classification (IPC) or to both national classification and IPC

**B. FIELDS SEARCHED**

Minimum documentation searched (classification system followed by classification symbols)

C12N1/20; C12Q1/06; G01N27/48; C12M1/34

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2004-337029 A (ASTEC CO., LTD.) 02 December 2004 (2004-12-02) claims 3-4, paragraphs [0020], [0022], fig. 1 | 2 |
| A | claims 3-4, paragraphs [0020], [0022], fig. 1 | 1, 3-4 |
| X | ISHIKI, K. et al., Electrochemical detection of viable bacterial cells using a tetrazolium salt, Anal. Chem., 2018, vol. 90, pp. 10903-10909 abstract, fig. 2 | 4 |
| A | abstract, fig. 2 | 1-3 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 July 2023** | **18 July 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/016896**

| Patent document cited in search report | | | Publication date (day/month/year) | Patent family member(s) | Publication date (day/month/year) |
|---|---|---|---|---|---|
| JP | 2004-337029 | A | 02 December 2004 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4448223 B

**Non-patent literature cited in the description**

- **KENGO ISHIKI ; DUNG Q. NGUYEN ; AYA MORISHITA ; HIROSHI SHIIGI ; TSUTOMU NA-GAOKA**. Electrochemical Detection of Viable Bacterial Cells Using a Tetrazolium Salt. *Analytical Chemistry*, 2018, vol. 90, 903-909